**Europäisches Patentamt**

**European Patent Office**

**Office européen des brevets**

(19)

(11) Veröffentlichungsnummer: **0 261 387 B1**

(12) ## EUROPÄISCHE PATENTSCHRIFT

(45) Veröffentlichungstag der Patentschrift: **08.05.91**

(51) Int. Cl.⁵: **A61K 7/09**

(21) Anmeldenummer: **87111916.0**

(22) Anmeldetag: **18.08.87**

(54) **Mittel und Verfahren zur dauerhaften Haarverformung.**

(30) Priorität: **20.09.86 DE 3631991**

(43) Veröffentlichungstag der Anmeldung:
**30.03.88 Patentblatt 88/13**

(45) Bekanntmachung des Hinweises auf die
Patenterteilung:
**08.05.91 Patentblatt 91/19**

(84) Benannte Vertragsstaaten:
**AT BE CH DE ES FR GB GR IT LI NL SE**

(56) Entgegenhaltungen:
**FR-A- 1 367 854**
**JP-A- 5 762 217**

(73) Patentinhaber: **Wella Aktiengesellschaft**
**Berliner Allee 65**
**W-6100 Darmstadt(DE)**

(72) Erfinder: **Hartmann, Peter**
**Hoffmannstrasse 6**
**W-6100 darmstadt(DE)**
Erfinder: **Köhler, Joachim, Dr.**
**Im Trappengrund 1A**
**W-6107 Reinheim(DE)**
Erfinder: **Konrad, Eugen**
**Mecklenburger Strasse 101**
**W-6100 Darmstadt(DE)**

**Beschreibung**

Die vorliegende Erfindung betrifft ein aus mehreren Komponenten erhältliches Mittel sowie ein Verfahren zur dauerhaften Haarverformung.

Zur Vermeidung einer weiteren Haarschädigung werden für die Verformung von stark geschädigtem, gebleichtem oder gefärbtem Haar bevorzugt schwach sauer bis neutral eingestellte Verformungsmittel verwendet. Hierbei haben sich im Verlauf der letzten 30 Jahre die Thioglykolsäureester als für diesen Zweck am besten geeignete Reduktionsmittel erwiesen.

Dem Vorteil einer haarschonenderen Verformungsbehandlung durch schwach saure bis neutrale Verformungsmittel stehen jedoch eine Reihe von Nachteilen gegenüber. So besitzen diese Mittel eine im Vergleich zu mildalkalischen Verformungsmitteln auf Thioglykolatbasis geringere Wellwirksamkeit. Aus diesem Grunde ist für die Erzielung einer ausreichenden Umformung die Zufuhr von Wärme, eine Verlängerung der Einwirkungszeiten auf 20 bis 60 Minuten sowie die Verwendung von vergleichsweise dünnen Wicklern erforderlich. Eine Verwendung dieser Verformungsmittel für normales, nicht geschädigtes Naturhaar erscheint aufgrund der auch bei Wärmezufuhr erforderlichen langen Einwirkungszeiten von über 30 Minuten nicht sinnvoll, so daß die Anwendung von schwach sauer bis neutral eingestellten Verformungsmitteln bisher in der Regel auf vorgeschädigtes, leicht verformbares Haar beschränkt blieb.

Versuche, die Wirkung derartiger Präparate auf Thioglykolsäureester-Basis durch Zusatz bestimmter Mercaptoverbindungen, wie zum Beispiel Thioglykolsäure, Thiomilchsäure, Thioglycerin und Cystein, zu steigern, führten nicht zu der gewünschten Verbesserung sondern eher zu einer Verschlechterung des Wellergebnisses.

Es wurde nun gefunden, daß die Wirkung von Verformungsmitteln auf Thioglykolsäureester-Basis durch den Zusatz von Cysteamin verstärkt werden kann.

Aus der Literatur, beispielsweise der **EP-OS 0 095 916,** ist es bereits bekannt, Cysteamin in stark alkalischen Depilationsmitteln auf der Basis von bestimmten Mercaptoverbindungen, wie zum Beispiel Thioglykolsäure, Thiomilchsäure oder beta-Mercaptopropionsäure, zur Wirkungssteigerung einzusetzen. Ebenfalls ist aus der **offengelegten japanischen Patentanmeldung 57-62217** bekannt, daß ein Zusatz von Cysteamin zu für die Verwendung in Haarverformungsmitteln üblichen Reduktionsmitteln, wie beispielsweise Thioglykolsäure, Thioglycerin, Thiomilchsäure, Natriumhyposulfit oder Natriumsulfit, insbesondere im alkalischen pH-Bereich, eine synergistische Steigerung der Wellwirksamkeit bewirkt.

Eine Verwendung von Cysteamin zur Erhöhung der Verformungswirkung von sauren oder neutralen Verformungsmitteln, insbesondere solchen auf der Basis von Thioglykolsäureestern, wurde jedoch bisher nicht vorgeschlagen. In der Literatur (vergleiche die vorstehend genannte offengelegte japanische Patentanmeldung 57-62217) wird vielmehr ausdrücklich darauf hingewiesen, daß zur Erzielung eines guten Welleffektes die Verformungsbehandlung unter alkalischen Bedingungen erfolgen sollte.

Gegenstand der vorliegenden Erfindung ist daher ein Mittel zur dauerhaften Verformung der Haare auf der Basis eines Thioglykolsäureesters, welches dadurch gekennzeichnet ist, daß es Cysteamin oder dessen Salz enthält.

Während der Thioglykolsäureester, welcher vorzugsweise der Thioglykolsäuremonoglycerinester ist, in dem Verformungsmittel insbesondere in einer Menge von etwa 5 bis 30 Gewichtsprozent (entsprechend einem Gehalt an freier Thioglykolsäure von 2 bis 16 Gewichtsprozent) enthalten ist, beträgt der Gehalt an Cysteamin oder dessen Salz, insbesondere Cysteamin-Hydrochlorid, vorzugsweise etwa 0,01 bis 10 Gewichtsprozent.

Das erfindungsgemäße Verformungsmittel besitzt vorzugsweise einen pH-Wert von 4,5 bis 7,5.

Es kann sowohl in Form einer wäßrigen Lösung oder einer Emulsion als auch in verdickter Form auf wäßriger Basis, insbesondere als Gel, Creme oder Paste, vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,5 bis 20 Gewichtsprozent in diesem Mittel enthalten sein können.

Weiterin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von etwa 2 bis 30 Gewichtsprozent zugesetzt werden.

Da die Thioglykolsäureester in wäßrigem Medium nur kurze Zeit (maximal 8 bis 12 Stunden) beständig sind und, insbesondere im alkalischen Bereich, leicht hydrolysieren, ist es erforderlich, den Thioglykolsäureester in wasserfreier Form abzupacken und das Verformungsmittel vor Gebrauch durch Vermischen der thioglykolsäureesterhaltigen Komponente mit einer oder mehreren weiteren Komponenten herzustellen.

Je nach Konfektionierung kann das erfindungsgemäße Mittel hierbei in Form eines Zwei- oder Dreikomponenten-Präparates vorliegen.

So ist das erfindungsgemäße Mittel zum Beispiel durch Vermischen zweier Komponenten erhältlich, von denen die erste Komponente das Cysteamin oder dessen Salz sowie Wasser enthält, während die zweite, wasserfreie Komponente den Thioglykolsäureester enthält.

In einer weiteren Ausführungsform wird das erfindungsgemäße Mittel durch Vermischen einer wasserfreien, den Thioglykolsäureester und das Cysteamin oder dessen Salz enthaltenden Komponente mit Wasser hergestellt.

Ebenfalls ist es möglich, das erfindungsgemäße Mittel in Form eines Dreikomponenten-Präparates abzupacken, wobei eine Komponente das Cysteamin oder dessen Salz, eine zweite, wasserfreie Komponente den Thioglykolsäureester und die dritte Komponente die wäßrige Phase enthält.

Bei allen Ausführungsformen des erfindungsgemäßen Mittels können die vorstehend genannten kosmetischen Zusatzstoffe sowohl in der wäßrigen als auch in der/den nichtwäßrigen Komponente(n) enthalten sein, wobei die nicht-wäßrigen Komponenten sowohl als Flüssigkeit als auch in fester Form, beispielsweise als Pulver, vorliegen können.

Durch die synergistische Wirkung der Kombination aus Thioglykolsäureester und Cysteamin beziehungsweise dessen Salz wird ein saures bis pH-neutrales Haarverformungsmittel auf Thioglykolsäureester-Basis zur Verfügung gestellt, das universell für jede Haarstruktur, gegebenenfalls unter zusätzlicher Wärmeeinwirkung, geeignet ist. Das Mittel bewirkt eine elastische, gleichmäßige Umformung vom Haaransatz bis zu den Haarspitzen, ohne die vorgeschädigten Haarspitzen zu überkrausen, wie dies bei mildalkalischen Präparaten häufig der Fall ist.

Weiterhin werden durch das erfindungsgemäße Mittel die Einwirkungszeiten gegenüber bisher üblichen Verformungsmitteln auf Thioglykolsäureesterbasis erheblich verkürzt, so daß eine Umformung auch schwer verformbaren Haares unter Anwendung von Wärme (vorzugsweise bei einer Temperatur von 30 bis 55 Grad Celsius) in etwa 15 bis 20 Minuten gelingt. Ebenfalls ermöglichen die erfindungsgemäßen Mittel auf der Basis eines Thioglykolsäureesters eine Verformung von leicht wellbarem Haar ohne Wärmeeinwirkung in etwa 20 Minuten sowie die Verwendung größerer Wickler, deren Durchmesser dem der für die Haarverformung mit mildalkalischen Mitteln gebräuchlichen Wickler entspricht.

Die vorliegende Erfindung betrifft daher weiterhin ein Verfahren zur dauerhaften Verformung von Haaren, bei dem man das Haar bevor und/oder nachdem man es in die gewünschte Form bringt mit einem Verformungsmittel behandelt, gegebenenfalls mit Wasser spült, sodann oxidativ nachbehandelt, mit Wasser spült, gegebenenfalls zur Wasserwelle legt und sodann trocknet, welches dadurch gekennzeichnet ist, daß für die Verformungsbehandlung das vorstehend beschriebene Verformungsmittel verwendet wird.

Bei einer Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von etwa 5 bis 15 Millimetern gewickelt. Sodann wird das Haar mit einer für die Haarverformung ausreichenden Menge, vorzugsweise etwa 80 bis 120 Gramm, des beschriebenen erfindungsgemäßen Verformungsmittels behandelt.

In einer weiteren Ausführungsform des erfindungsgemäßen Verfahrens wird das Haar im Anschluß an die Haarwäsche mit einem Teil des vorstehend beschriebenen erfindungsgemäßen Verformungsmittels, vorzugsweise etwa 40 bis 60 Gramm, vorgefeuchtet, in einzelne Strähnen aufgeteilt und auf Wickler gewickelt. Sodann wird das Haar mit dem restlichen Verformungsmittel, vorzugsweise etwa 40 bis 60 Gramm, nochmals behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 5 bis 30 Minuten beträgt, wird das Haar mit Wasser gespült und dann oxidativ nachbehandelt ("fixiert"). Das Nachbehandlungsmittel wird, je nach Haarfülle, in einer Menge von etwa 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel etwa 5 bis 15 Minuten) und der Anwendungstemperatur unterschiedlich. Normalerweise liegt das Oxidationsmittel in den gebrauchsfertigen wäßrigen Nachbehandlungsmitteln in einer Konzentration von etwa 0,5 bis 10 Gewichtsprozent vor. Das Mittel für die oxidative Nachbehandlung kann selbstverständlich weitere Stoffe, wie zum Beispiel Netzmittel, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung erläutern, ohne ihn hierauf zu beschränken.

**Beispiele**

**Beispiel 1**

|  | | |
|---|---|---|
| Komponente 1: | 0,20 g | Cysteamin |
| | 0,30 g | Parfümöl |
| | 99,50 g | Wasser, vollentsalzt |
| | 100,00 g | |

|  | | |
|---|---|---|
| Komponente 2: | 60,00 g | Thioglykolsäuremonoglycerinester |
| | 40,00 g | Glycerin |
| | 100,00 g | |

Vor dem Gebrauch werden 80 Gramm der Komponente 1 und 40 Gramm der Komponente 2 miteinander vermischt. Der pH-Wert dieses Haarverformungsmittels bet ragt 6,0.

Leicht vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 15 Minuten lang unter einer Trockenhaube bei einer Temperatur von 40 Grad Celsius erwärmt . Anschließend wird die Abdeckung entfernt, das Haar mit Wasser gespült und mit 100 Gramm einer 3-prozentigen wäßrigen Hydrogenperoxidlösung oxidativ nachbehandelt . Nach Entfernung der Wickler werden die Haare erneut mit Wasser gespült, zur Wasserwelle gelegt und sodann getrocknet.

Als Ergebnis dieser Behandlung wird eine gleichmäßige, natürlich wirkende Umformung der Haare vom Haaransatz bis zu den Haarspitzen erhalten.

**Beispiel 2**

```
Komponente 1:      1,00 g    Cysteamin
                   1,00 g    Cysteamin-Hydrochlorid
                   0,30 g    Parfümöl
                  97,70 g    Wasser, vollentsalzt
                 ─────────
                 100,00 g
```

```
Komponente 2:     60,00 g    Thioglykolsäuremonoglycerinester
                  40,00 g    Glycerin
                 ─────────
                 100,00 g
```

Vor dem Gebrauch werden 60 Gramm der Komponente 1 und 30 Gramm der Komponente 2 zu einem Haarverformungsmittel mit einem pH-Wert von 6,6 vermischt.

Leicht verformbares Haar wird mit einem Shampoo gewaschen und mit Wasser gründlich gespült. Anschließend wird etwa die Hälfte des Haarverformungsmittels gleichmäßig auf dem frottierten Haar verteilt, das Haar auf Wickler mit einem Durchmesser von 10 Millimetern gewickelt und mit dem restlichen Haarverformungsmittel nochmals befeuchtet. Nach einer Einwirkungszeit von 20 Minuten wird das Haar mit Wasser gespült und mit 100 Gramm einer 2-prozentigen Hydrogenperoxidlösung oxidativ nachbehandelt . Nach Entfernung der Wickler werden die Haare erneut mit Wasser gespült und danach getrocknet.

**Beispiel 3**

```
Komponente 1:     10,00 g    Cysteamin-Hydrochlorid
                   1,50 g    Ammoniak, 25-prozentige wäßrige
                             Lösung
                   0,30 g    Parfümöl
                  88,20 g    Wasser, vollentsalzt
                 ─────────
                 100,00 g
```

```
Komponente 2:     60,00 g    Thioglykolsäuremonoglycerinester
                  40,00 g    Glycerin
                 ─────────
                 100,00 g
```

80 Gramm der Komponente 1 werden mit 40 Gramm der Komponente 2 zu einem gebrauchsfertigen Haarverformungsmittel vom pH 6,5 vermischt.

Normales, nicht vorgeschädigtes und daher schwer verformbares Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 20 Minuten wird das Haar mit Wasser gründlich gespült und sodann mit 80 Gramm einer 3-prozentigen Hydrogenperoxidlösung oxidativ nachbehandelt. Nach Entfernung der Wickler werden die Haare erneut mit Wasser gespült, zur Wasserwelle gelegt und anschließend getrocknet.

Das so umgeformte Haar besitzt eine gleichmäßige Krause, die mit einer durch Behandlung mit mildalkalischen Dauerverformungsmitteln erzielbaren Krause vergleichbar ist.

**Beispiel 4**

```
        Komponente 1:          10,00 g    Cysteamin-Hydrochlorid

        Komponente 2:           1,50 g    Ammoniak, 25-prozentige
                                           wäßrige Lösung
                                0,30 g    Parfümöl
                               88,20 g    Wasser, vollentsalzt
                              _____

        Komponente 1 + 2:  100,00 g

        Komponente 3:          60,00 g    Thioglykolsäuremonoglycerin-
                                           ester
                               40,00 g    Glycerin
                              _____

              - -            100,00 g
```

Komponente 1 wird in Komponente 2 gelöst. Anschließend werden 80 Gramm dieser Lösung mit 40 Gramm der Komponente 3 vermischt. Der pH-Wert des gebrauchsfertigen Haarverformungsmittels beträgt 6,5.

Normales, nicht vorgeschädigtes Haar wird im Anschluß an die Haarwäsche mit der Hälfte des vorstehend beschriebenen Haarverformungsmittels vorgefeuchtet und auf Wickler mit einem Durchmesser von 8 Millimetern gewickelt. Sodann wird das Haar mit dem restlichen Verformungsmittel nochmals behandelt und mit einem Wärmestrahler 15 Minuten erwärmt. Anschließend wird das Haar mit Wasser gespült und mit 100 Gramm einer 3-prozentigen wäßrigen Hydrogenperoxidlösung oxidativ nachbehandelt. Nach der Entfernung der Wickler werden die Haare erneut mit Wasser gespült und getrocknet.

Als Thioglycolsäuremonoglycerinester wurde ein handelsüblicher Thioglykolsäureglycerinester verwendet. Der pH-Wert einer 20-prozentigen wäßrigen Lösung dieses Thioglykolsäureglycerinesters beträgt etwa 2 bis 3.

Alle in der vorliegenden Anmeldung enthaltenen Prozentangaben stellen Gewichtsprozente dar.

**Ansprüche**

1. Mittel zur dauerhaften Verformung von Haaren auf der Basis eines Thioglykolsäureesters, dadurch gekennzeichnet, daß es Cysteamin und/oder dessen Salz enthält.

2. Mittel nach Anspruch 1, dadurch gekennzeichnet, daß es 0,01 bis 10 Gewichtsprozent Cysteamin oder dessen Salz enthält.

3. Mittel nach Anspruch 1 und 2 dadurch gekennzeichnet, daß das Cysteaminsalz das Cysteamin-Hydrochlorid ist.

4. Mittel nach Anspruch 1 bis 3, dadurch gekennzeichnet, daß der Thioglykolsäureester in einer Menge von 5 bis 30 Gewichtsprozent enthalten ist.

5. Mittel nach Anspruch 1 bis 4, dadurch gekennzeichnet, daß der Thioglykolsäureester der Thioglykolsäuremonoglycerinester ist.

6. Mittel nach Anspruch 1 bis 5, dadurch gekennzeichnet, daß der pH-Wert 4,5 bis 7,5 beträgt.

7. Mittel nach Anspruch 1 bis 6, dadurch gekennzeichnet, daß es vor der Anwendung durch Vermischen mehrerer Komponenten, wobei die thioglykolsäureesterhaltige Komponente wasserfrei ist, hergestellt wird.

8. Mittel nach Anspruch 7, dadurch gekennzeichnet, daß es durch Vermischen von zwei Komponenten hergestellt wird, wobei die erste Komponente Cysteamin oder dessen Salz und Wasser enthält, während die zweite Komponente den Thioglykolsäureester enthält.

9. Mittel nach Anspruch 7, dadurch gekennzeichnet, daß die erste Komponente den Thioglykolsäureester und Cysteamin oder dessen Salz enthält, während die zweite Komponente Wasser enthält.

10. Mittel nach Anspruch 7, dadurch gekennzeichnet, daß es durch Vermischen von drei Komponenten hergestellt wird, wobei die erste Komponente Cysteamin oder dessen Salz, die zweite Komponente einen Thioglykolsäureester und die dritte Komponente die wäßrige Phase enthält.

11. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, dadurch gekennzeichnet, daß man als Verformungsmittel ein Mittel nach Anspruch 1 bis 10 verwendet.

12. Verfahren nach Anspruch 11, dadurch gekennzeichnet, daß man das Verformungsmittel 5 bis 30 Minuten lang einwirken läßt.

13. Verfahren nach Anspruch 11, dadurch gekennzeichnet, daß man das Verformungsmittel unter Anwendung von Wärme (30-55° C) 15 bis 20 Minuten lang einwirken läßt.

14. Verfahren nach Anspruch 11 bis 13, dadurch gekennzeichnet, daß man das Verformungsmittel in einer Menge von 80 bis 120 Gramm anwendet.

## Claims

1. Agent for the permanent shaping of hair, based on a thioglycollic acid ester, characterised in that it contains cysteamine and/or a salt thereof.

2. Agent according to claim 1, characterised in that it contains 0.01 to 10% by weight of cysteamine or a salt thereof.

3. Agent according to claim 1 and 2, characterised in that the cysteamine salt is cysteamine hydrochloride.

4. Agent according to claim 1 to 3, characterized in that the thioglycollic acid ester is present in a quantity of 5 to 30% by weight.

5. Agent according to claim 1 to 4, characterized in that the thioglycollic acid ester is thioglycollic acid monoglycerol ester.

6. Agent according to claim 1 to 5, characterized in that its pH is 4.5 to 7.5.

7. Agent according to claim 1 to 6, characterised in that prior to use, it is made up by blending a plurality of components, wherein the component containing thioglycollic acid ester is anhydrous.

8. Agent according to claim 7, characterised in that it is produced by blending two components, the first component contain cysteamine or a salt thereof and water, while the second component contains the thioglycollic acid ester.

9. Agent according to claim 7, characterised in that the first component contains the thioglycollic acid ester and cysteamine or a salt thereof, while the second component contains water.

10. Agent according to claim 7, characterised in that it is produced by blending three components, wherein the first component contains cysteamine or a salt thereof, the second component contains a thioglycollic acid ester and the third component contains the aqueous phase.

11. A method of permanently shaping hair, in which before and/or after the hair is styled as desired, it is treated with a shaping agent, rinsed with water and then given a subsequent oxidative treatment, rinsed with water, possibly set with a water wave and then dried, characterised in that the shaping agent used is an agent according to claim 1 to 10.

12. A method according to claim 11, characterized in that the shaping agent is allowed to act for 5 to 30 minutes.

13. A method according to claim 11, characterized in that the shaping agent is allowed to act for 15 to 20 minutes with the application of heat (30-55° C).

14. Method according to claim 11 to 13, characterised in that the shaping agent is used in a quantity of 80 to 120g.

## Revendications

1. Produit pour déformer les cheveux de façon permanente, à base d'un ester thioglycolique, caractérisé en ce qu'il contient de la cystéamine ou/et son sel.

2. Produit selon la revendication 1, caractérisé en ce qu'il contient 0,01 à 10 % en poids de cystéamine ou de son sel.

3. Produit selon la revendication 1 ou 2, caractérisé en ce que le sel de cystéamine est le chlorure de cystéamine.

4. Produit selon les revendications 1 à 3, caractérisé en ce que l'ester thioglycolique est contenu en une quantité allant de 5 à 30 % en poids.

5. Produit selon les revendications 1 à 4, caractérisé en ce que l'ester thioglycolique est le monoglycérate d'acide thioglycolique.

6. Produit selon les revendications 1 à 5, caractérisé en ce que le pH est compris entre 4,5 et 7,5.

7. Produit selon les revendications 1 à 6, caractérisé en ce qu'il est préparé en mélangeant, avant utilisation, plusieurs composants, le composant contenant l'ester thioglycolique étant anhydre.

8. Produit selon la revendication 7, caractérisé en ce qu'il est préparé en mélangeant deux composants, le premier composant contenant de la cystéamine ou son sel et de l'eau, tandis que le deuxième composant contient l'ester thioglycolique.

9. Produit selon la revendication 7, caractérisé en ce que le premier composant contient l'ester thioglycolique et la cystéamine ou son sel, tandis que le deuxième composant contient de l'eau.

10. Produit selon la revendication 7, caractérisé en ce qu'il est préparé en mélangeant trois composants, le premier composant contenant la cystéamine ou son sel, le deuxième composant comportant un ester thioglycolique et le troisième composant la phase aqueuse.

11. Procédé pour déformer les cheveux de façon durable, dans lequel, avant qu'on ait conféré au cheveu la forme souhaitée, ou après, on traite le cheveu avec un produit de déformation, on le rince à l'eau, on le traite ensuite avec un oxydant, on le rince à l'eau, on le met éventuellement en plis, puis on le sèche, caractérisé en ce qu'on utilise comme produit de déformation un produit selon les revendications 1 à 10.

12. Procédé selon la revendication 11, caractérisé en ce qu'on laisse agir le produit de déformation 5 à 30 minutes.

13. Procédé selon la revendication 11, caractérisé en ce qu'on laisse agir le produit de déformation 15 à 20 minutes en utilisant la chaleur (30-55° C).

14. Procédé selon les revendications il à 13, caractérisé en ce qu'on utilise le produit de déformation en une quantité allant de 80 à 120 grammes.